# EUROPEAN PATENT APPLICATION

(11) **EP 4 850 741 A1**
(43) Date of publication of application: **09.12.2026**
(21) Application number: 24921178.0
(22) Date of filing: 16.04.2024
(51) Int. Cl.: A61M 35/00, B05B 15/00

(54) **FLIP COVER MECHANISM AND ATOMIZATION DEVICE**

(30) Priority: 31.01.2024 CN 202410142398
(71) Applicant: Shenzhen Moore Vaporization Health & Medical Technology Co., Ltd., Shenzhen, Guangdong 518102 (CN)
(72) Inventor: ZHAO, Tao, Shenzhen, Guangdong 518102 (CN); FENG, Ke, Shenzhen, Guangdong 518102 (CN); ZHENG, Xin, Shenzhen, Guangdong 518102 (CN)
(74) Representative: Winter, Brandl - Partnerschaft mbB
(86) International application number: PCT/CN2024/088046
(87) International publication number: WO 2025/161134

(57) **Abstract**

This application provides a flip cover mechanism and an atomization apparatus, to resolve a problem of low efficiency of atomization mechanism replacement in the related art. The flip cover mechanism includes a flip cover and a limiting mechanism. The limiting mechanism has a limiting state and a released state. When the flip cover is rotated, linkage engagement between the flip cover and the limiting mechanism is discontinuous coupling, to cause the flip cover to switch a state of the limiting mechanism.

## Description

### CROSS-REFERENCE TO RELATED APPLICATIONS

This application claims priority to Chinese Patent Application No. 2024101423986, filed on January 31, 2024, which is incorporated herein by reference in its entirety.

### TECHNICAL FIELD

This application relates to the field of atomization device technologies, and in particular, to a flip cover mechanism and an atomization apparatus.

### BACKGROUND

A cosmetic device sprays cosmetic liquid onto, for example, the skin, so as to enable better absorption of the cosmetic liquid by the skin. The cosmetic device includes a housing, an atomization mechanism (configured to spray the cosmetic device), and a cover plate. Upon detachment of the atomization mechanism, the cover plate is first opened, and then a component (for example, a bolt) that limits the atomization mechanism is removed, so that the atomization mechanism is detached from the housing. This results in a large quantity of steps for detaching the atomization mechanism and low efficiency of atomization mechanism replacement.

### SUMMARY

This application mainly resolves a technical problem by providing a flip cover mechanism and an atomization apparatus, to resolve a problem of low efficiency of atomization mechanism replacement in the related art.

To resolve the foregoing technical problem, a first technical solution used in this application is as follows. A flip cover mechanism is provided. The flip cover mechanism includes a flip cover and a limiting mechanism. The limiting mechanism has a limiting state and a released state. When the flip cover is rotated, linkage engagement between the flip cover and the limiting mechanism is discontinuous coupling, to cause the flip cover to switch a state of the limiting mechanism. In this way, the discontinuous coupling can avoid that the flip cover is always coupled to the limiting mechanism when the flip cover is rotated (for example, in a process of opening or closing the flip cover), and can cause more operation manners (for example, a cover-opening free stroke and a cover-opening coupling stroke below) between the flip cover and the limiting mechanism. Switching the state of the limiting mechanism can cause the limiting mechanism to fasten an atomization mechanism in a housing, or release a limitation on the atomization mechanism, to facilitate mounting and detaching of the atomization mechanism. Through engagement between the discontinuous coupling and the state switching of the limiting mechanism, the step of separately operating the limiting mechanism to release or limit the atomization mechanism can be omitted, to resolve a problem that a separate operation step for the limiting mechanism is still required after the flip cover is rotated, thereby improving efficiency of atomization mechanism replacement.

In some embodiments, in a process of opening the flip cover, linkage engagement between the flip cover and the limiting mechanism is discontinuous coupling, to cause the flip cover to switch the state of the limiting mechanism from the limiting state to the released state.

In some embodiments, the process of opening the flip cover sequentially includes a cover-opening idle stroke and a cover-opening coupling stroke. During the cover-opening idle stroke, the flip cover is decoupled from the limiting mechanism, and the limiting mechanism remains in the limiting state. During the cover-opening coupling stroke, the flip cover is coupled to the limiting mechanism, to cause the flip cover to switch the state of the limiting mechanism to the released state.

In some embodiments, the limiting mechanism includes a rotating assembly and an elastic assembly, where the elastic assembly is connected to the rotating assembly. During the cover-opening idle stroke, the flip cover is decoupled from the rotating assembly, and the elastic assembly drives the rotating assembly to be in the limiting state.

During the cover-opening coupling stroke, the flip cover is coupled to the rotating assembly, to cause the state of the rotating assembly to be switched to the released state, and the elastic assembly is configured to force the rotating assembly to reset to the limiting state.

In some embodiments, the rotating assembly includes a rotating member and a sliding member; and the elastic assembly includes a second elastic member and a third elastic member. The second elastic member is connected to the rotating member, and the third elastic member is connected to the sliding member. During the cover-opening idle stroke, the flip cover is decoupled from the rotating member, and the third elastic member drives the sliding member to be in the limiting state; and the rotating member is decoupled from the sliding member, or the second elastic member drives the rotating member and the sliding member to be in a balanced state. During the cover-opening coupling stroke, the flip cover is coupled to the rotating member, to cause the rotating member to abut against the sliding member, the rotating member drives the sliding member to rotate to be in the released state, and both an elastic force of the second elastic member and an elastic force of the third elastic member increase.

In some embodiments, the process of opening the flip cover further includes a cover-opening free stroke after the cover-opening coupling stroke. During the cover-opening free stroke, the flip cover is decoupled from the limiting mechanism, and the limiting mechanism remains in the released state.

In some embodiments, in a process of closing the flip cover, linkage engagement between the flip cover and the limiting mechanism is decoupling, and the state of the limiting mechanism remains in the limiting state.

In some embodiments, in the process of closing the flip cover, the linkage engagement between the flip cover and the limiting mechanism is discontinuous coupling, to cause the flip cover to switch the state of the limiting mechanism from the released state to the limiting state.

In some embodiments, the process of closing the flip cover sequentially includes a cover-closing free stroke, a cover-closing coupling stroke, and a cover-closing idle stroke. During the cover-closing free stroke, the flip cover is decoupled from the limiting mechanism, and the state of the limiting mechanism is the released state. During the cover-closing coupling stroke, the flip cover is coupled to the limiting mechanism, and the state of the limiting mechanism is the released state. During the cover-closing idle stroke, the flip cover is decoupled from the limiting mechanism, and the flip cover is configured to press the atomization mechanism, to cause the state of the limiting mechanism to be switched to the limiting state.

To resolve the foregoing technical problem, a second technical solution used in this application is as follows. An atomization apparatus is provided. The atomization apparatus includes a housing, an atomization mechanism, and the foregoing flip cover mechanism. The housing has an accommodating cavity and a mounting opening. The atomization mechanism is detachably mounted in the accommodating cavity. The flip cover is rotatably connected to the housing, and the flip cover is configured to cover the mounting opening; and the limiting mechanism is movably disposed in the housing.

In some embodiments, the limiting mechanism is in insertion engagement with a limiting portion of the atomization mechanism; the limiting mechanism includes the sliding member, and the limiting portion is a groove; and in the limiting state, at least a part of the sliding member is inserted in the groove.

In some embodiments, the atomization mechanism includes an atomization assembly and an atomization cartridge that are detachably connected; and an outer wall of the atomization assembly has a groove; or an outer wall of the atomization cartridge has a groove; or a groove is formed between an outer bottom wall of the atomization assembly and an outer top wall of the atomization cartridge.

In some embodiments, the atomization apparatus further includes a first elastic member, configured to apply an elastic force to the atomization mechanism, to cause the limiting mechanism to abut against a non-limiting portion of the atomization mechanism during the transition from the cover-opening coupling stroke to the cover-opening free stroke.

### BRIEF DESCRIPTION OF THE DRAWINGS

To describe technical solutions of embodiments of this application more clearly, the following briefly introduces the accompanying drawings required for describing the embodiments. Apparently, the accompanying drawings in the following descriptions merely show some embodiments of this application, and a person of ordinary skill in the art may still derive other accompanying drawings from the accompanying drawings without creative efforts.
FIG. 1 is a schematic structural diagram of an atomization device according to this application;
FIG. 2 is a schematic structural diagram of a state of an atomization apparatus according to this application; FIG. 3 is an enlarged view of Q in FIG. 2;
FIG. 4 is a schematic structural diagram of another state of an atomization apparatus according to this application; FIG. 5 is an enlarged view of P in FIG. 4;
FIG. 6 is a schematic structural diagram of another state of an atomization apparatus according to this application; FIG. 7 is an enlarged view of W in FIG. 6;
FIG. 8 is a schematic structural diagram of still another state of an atomization apparatus according to this application; FIG. 9 is an enlarged view of R in FIG. 8;
FIG. 10 is a schematic structural diagram of an atomization mechanism in an ejected state in an atomization apparatus according to this application;
FIG. 11 is a schematic structural diagram of an atomization apparatus according to this application, with a part of a housing removed;
FIG. 12 is a schematic structural diagram of an atomization apparatus according to this application, with an entire housing removed;
FIG. 13 is a schematic structural diagram of an atomization mechanism and a housing according to this application;
FIG. 14 is a schematic structural diagram of an atomization mechanism according to this application; and
FIG. 15 is a schematic structural diagram of an atomization mechanism according to this application.

In the accompanying drawings: 1: Charger; 2: Atomization apparatus; 20: First elastic member; 21: Flip cover; 211: Cover body; 212: Trigger portion; 22: Atomization mechanism; 221: Atomization assembly; 222: Atomization cartridge; 223: Groove; 224: Air supply interface; 2241: Snap-fit groove; 225: Atomization cavity; 226: Piston; 227: First outer wall; 228: Second outer wall; 229: Third outer wall; 23: Housing; 231: Accommodating cavity; 232: Positioning groove; 24: Limiting mechanism; 241: Rotating assembly; 2411: Sliding member; 2412: Rotating member; 242: Elastic assembly; 2421: Third elastic member; 2422: Second elastic member; 25: Air supply mechanism; 26: Liquid pushing mechanism; 27: Circuit board; 28: Power supply; and 29: First sensor.

### DETAILED DESCRIPTION

The following describes solutions of embodiments of this application in detail with reference to the accompanying drawings of this specification.

In the following descriptions, for the purpose of illustration rather than limitation, specific details such as a specific system structure, interface, and technology are proposed to thoroughly understand this application.

The following clearly and completely describes the technical solutions in the embodiments of this application with reference to the accompanying drawings in the embodiments of this application. It is clear that the described embodiments are merely some but not all of the embodiments of this application. All other embodiments obtained by a person of ordinary skill in the art based on the embodiments of this application without creative efforts shall fall within the protection scope of this application.

The terms "first", "second", and "third" in this application are merely used for description, and cannot be understood as indicating or implying relative importance or implicitly indicating a quantity of indicated technical features. Therefore, a feature defined by "first", "second", or "third" may explicitly indicate or implicitly include at least one of such features. In the descriptions of this application, "a plurality of' means at least two, such as two or three, unless otherwise clearly specified. All directionality indications (for example, up, down, left, right, front, and back) in the embodiments of this application are merely used for explaining relative position relationships, movement situations or the like between various components in a specific posture (as shown in the accompanying drawings). If the specific posture changes, the directional indications change accordingly. In addition, the terms "include", "have", and any variants thereof are intended to cover a non-exclusive inclusion. For example, a process, method, system, product, or device that includes a series of steps or units is not limited to the listed steps or units, but optionally further includes unlisted steps or units, or optionally further includes another step or unit that is inherent to the process, method, product, or device.

The "embodiment" mentioned in this specification means that specific features, structures, or characteristics described with reference to the embodiments may be included in at least one embodiment of this application. The term appearing at various positions of this specification neither necessarily indicates same embodiments, nor indicates independent or alternative embodiments that are mutually exclusive to other embodiments. A person skilled in the art explicitly or implicitly understands that the embodiments described in this specification may be combined with other embodiments.

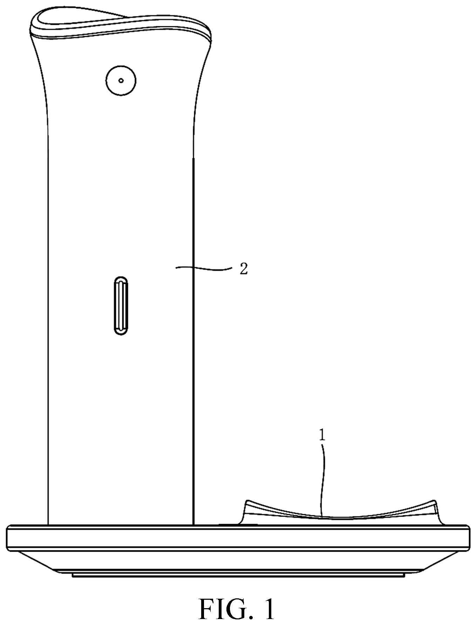

An embodiment of the present disclosure provides an atomization device. Refer to FIG. 1. The atomization device may include an atomization apparatus 2 and a charger 1. The charger 1 is configured to charge the atomization apparatus 2. In some examples, a charging port is provided on the atomization apparatus 2, and an insertion end of the charger 1 is inserted in the charging port, to charge a power supply 28 of the atomization apparatus 2.

In some other examples, the charger 1 charges the power supply 28 of the atomization apparatus 2 in a wireless manner. For example, the charger 1 charges the power supply 28 of the atomization apparatus 2 in a Bluetooth charging manner.

In some embodiments, the atomization device further includes another instrument. For example, the another instrument may be a standby charger 1 or a mounting box (where the mounting box is configured to assemble, for example, the atomization apparatus 2 and the charger 1 together, to facilitate transportation).

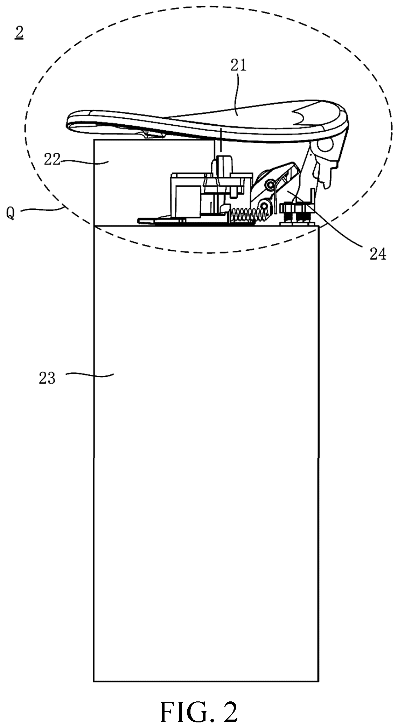

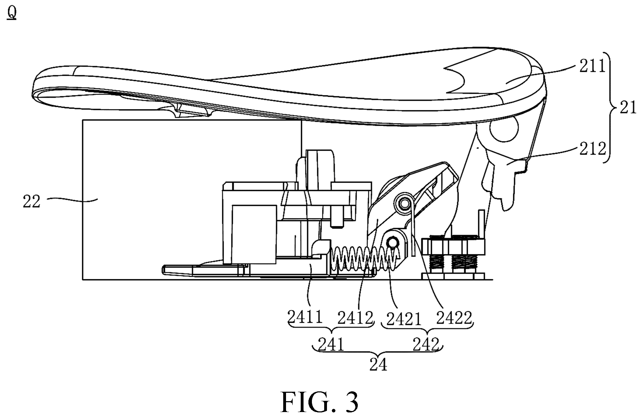

Refer to FIG. 2 and FIG. 3. The atomization apparatus may include a housing 23, an atomization mechanism 22, and a flip cover mechanism.

The housing 23 has an accommodating cavity 231 and a mounting opening. The mounting opening communicates with the accommodating cavity. In this way, the atomization mechanism 22 can be mounted in the accommodating cavity through the mounting opening. For example, a liquid pushing assembly below, an air supply mechanism 25 below, and the power supply 28 can all be mounted in the accommodating cavity through the mounting opening. For another example, the housing further has a receiving cavity. The receiving cavity also communicates with the mounting opening. The liquid pushing assembly below, the air supply mechanism 25 below, and the power supply 28 can all be mounted in the receiving cavity through the mounting opening. For another example, the housing further has a receiving cavity and an accommodating opening. The receiving cavity also communicates with the accommodating opening. The liquid pushing assembly below, the air supply mechanism 25 below, and the power supply 28 can all be mounted in the receiving cavity through the accommodating opening.

The atomization mechanism 22 is configured to spray an atomized liquid (which may be referred to as an atomized substrate). For example, the liquid may be a medicinal liquid, a nutrient solution, a cosmetic liquid, a serum, an essence emulsion, a disinfectant, an insecticide, a pesticide, or the like, so that the atomization apparatus 2 can be used in various fields (for example, the medical field, the cosmetic field, and the agricultural field). For example, when the atomization apparatus 2 is used in the cosmetic field, the atomization apparatus 2 may be referred to as a cosmetic device, an atomizer, or the like. The atomization mechanism 22 may include an atomization assembly 221 and an atomization cartridge 222 that are detachably connected (for example, snap-fitted). The atomization assembly 221 communicates with the atomization cartridge 222. The atomization cartridge 222 is configured to store a liquid (which may also be referred to as a substrate). The liquid in the atomization cartridge 222 flows to the atomization assembly 221, and is atomized and sprayed by the atomization assembly 221.

The atomization mechanism 22 is detachably mounted in the accommodating cavity 231. For example, the atomization mechanism 22 may be detachably mounted in the accommodating cavity through a snap, a bolt, a pin, or the like. For another example, a limiting mechanism 24 limits the atomization mechanism 22 in the accommodating cavity. In this way, during atomization mechanism replacement, the atomization mechanism 22 can be removed from the mounting opening, and then a new atomization mechanism 22 that needs to be replaced is mounted in the accommodating cavity through the mounting opening, thereby resolving a problem that it is not convenient to replace an atomization mechanism in an existing cosmetic device. A plurality of atomization mechanisms 22 may be provided. In this way, different cosmetic liquids may be respectively injected to the plurality of atomization mechanisms 22, to reduce microbial deterioration, contamination, and the like inside the atomization mechanism.

The flip cover mechanism may be used in a type of apparatus provided with a housing 23 having a mounting opening, and can be engaged with a to-be-mounted member, to mount the to-be-mounted member in the housing 23 or detach the to-be-mounted member from the housing 23. For example, the apparatus may be the atomization apparatus 2. In other words, the flip cover mechanism is used in the atomization apparatus 2. In this case, the to-be-mounted member may be the atomization mechanism 22. For another example, the apparatus may be a display screen, a main chassis, or the like. Descriptions are provided herein by using an example in which the flip cover mechanism may be used in the atomization apparatus 2, and the to-be-mounted member may be the atomization mechanism 22.

The flip cover mechanism may include a flip cover 21 and a limiting mechanism 24.

The flip cover 21 is configured to cover the mounting opening, and the mounting opening can be opened and closed by rotating the flip cover 21. A material of the flip cover 21 may be a metal material, for example, may be iron or copper; or may be a non-metal material, for example, may be plastic.

The flip cover 21 is rotatably connected to the housing 23. For example, the flip cover 21 is hingedly connected to the housing 23. The limiting mechanism 24 is movably disposed in the housing 23. In this way, linkage engagement can be formed between the limiting mechanism 24 and the flip cover 21, so that a state of the limiting mechanism 24 can be switched to a corresponding state through rotation of the flip cover 21.

The limiting mechanism 24 has a limiting state and a released state. The limiting state may be understood as a state in which the limiting mechanism 24 is configured to limit the atomization mechanism 22 in the housing 23. In other words, the limiting mechanism 24 is engaged with a limiting portion of the atomization mechanism 22, to limit the atomization mechanism 22 in the accommodating cavity.

The released state may be understood as a state in which the limiting mechanism 24 is configured to release the atomization mechanism 22. In other words, the flip cover 21 causes (for example, in a manner of driving, actuating, or triggering) the limiting mechanism 24 to release a limitation on the limiting portion. In other words, in this specification, the state of the limiting mechanism 24 is referred to as the limiting state only when the limiting mechanism 24 is engaged with the limiting portion of the atomization mechanism 22. Any state of the limiting mechanism 24 other than the limiting state may be referred to as the released state. For example, the released state of the limiting mechanism 24 may be a state of the limiting mechanism 24 when the limiting mechanism 24 is in contact with a non-limiting portion of the atomization mechanism 22. For another example, the released state may alternatively be a state of the limiting mechanism 24 when the limiting mechanism 24 is decoupled (which may be referred to as uncoupled or separated) from the atomization mechanism 22.

When the flip cover 21 is rotated, linkage engagement between the flip cover 21 and the limiting mechanism 24 is discontinuous coupling, to cause the flip cover 21 to switch a state of the limiting mechanism 24. In this way, the discontinuous coupling can avoid that the flip cover 21 is always coupled to the limiting mechanism 24 when the flip cover 21 is rotated (for example, in a process of opening or closing the flip cover 21), and can cause more operation manners (for example, a cover-opening free stroke and a cover-opening coupling stroke below) between the flip cover 21 and the limiting mechanism 24. Switching the state of the limiting mechanism 24 can cause the limiting mechanism 24 to fasten the atomization mechanism 22 in the housing 23, or remove a limitation on the atomization mechanism 22, to facilitate mounting and detaching of the atomization mechanism 22. Through engagement between the discontinuous coupling and the state switching of the limiting mechanism 24, the step of separately operating the limiting mechanism 24 to release or limit the atomization mechanism 22 can be omitted, to resolve a problem that a separate operation step for the limiting mechanism 24 is still required after the flip cover 21 is rotated,

The linkage engagement between the flip cover 21 and the limiting mechanism 24 being discontinuous coupling may be understood as follows: When the flip cover 21 is rotated, the rotation process may be divided into a decoupling stroke (for example, a cover-opening free stroke, a cover-opening idle stroke, a cover-closing free stroke, and a cover-closing idle stroke below) and a coupling stroke (for example, a cover-opening coupling stroke and a cover-closing coupling stroke below) between the flip cover 21 and the limiting mechanism 24.

The flip cover 21 switching the state of the limiting mechanism 24 may be understood as: switching the state of the limiting mechanism 24 from the limiting state to the released state, or switching the state of the limiting mechanism 24 from the released state to the limiting state.

Rotation of the flip cover 21 may include a process of opening the flip cover 21 and a process of closing the flip cover 21.

In some embodiments, in the process of opening the flip cover 21, linkage engagement between the flip cover 21 and the limiting mechanism 24 is discontinuous coupling, to cause the flip cover 21 to switch the state of the limiting mechanism 24 from the limiting state to the released state.

In this way, when the flip cover 21 is opened, the mounting opening can be exposed, and the limiting mechanism 24 can also release a limitation on the atomization mechanism 22, to remove the atomization mechanism 22.

For example, in the process of opening the flip cover 21, the process of opening the flip cover 21 sequentially includes a cover-opening coupling stroke and a cover-opening free stroke. In this way, immediately upon opening of the flip cover 21, the flip cover 21 can cause the limiting mechanism 24 to release the limitation on the atomization mechanism 22.

For another example, in the process of opening the flip cover 21, the process of opening the flip cover 21 sequentially includes a cover-opening idle stroke and a cover-opening coupling stroke. In this way, the flip cover 21 can cause the limiting mechanism 24 to release the limitation on the atomization mechanism 22 only after the flip cover 21 is opened for a period of time/stroke (that is, during the cover-opening idle stroke), so that the atomization mechanism 22 can be removed from the housing 23. When the flip cover 21 is in the cover-opening idle stroke, the limiting mechanism 24 is in the limiting state, that is, the limiting mechanism 24 still limits the atomization mechanism 22, to avoid an accident caused by the flip cover 21 being opened due to accidental touch of a user. In some embodiments, the process of opening the flip cover 21 further includes a cover-opening free stroke after the cover-opening coupling stroke.

The cover-opening free stroke is similar to the cover-opening idle stroke, during which the flip cover 21 and the limiting mechanism 24 are in a decoupled state.

The following describes the cover-opening idle stroke, the cover-opening coupling stroke, and the cover-opening free stroke of the flip cover 21 in detail.

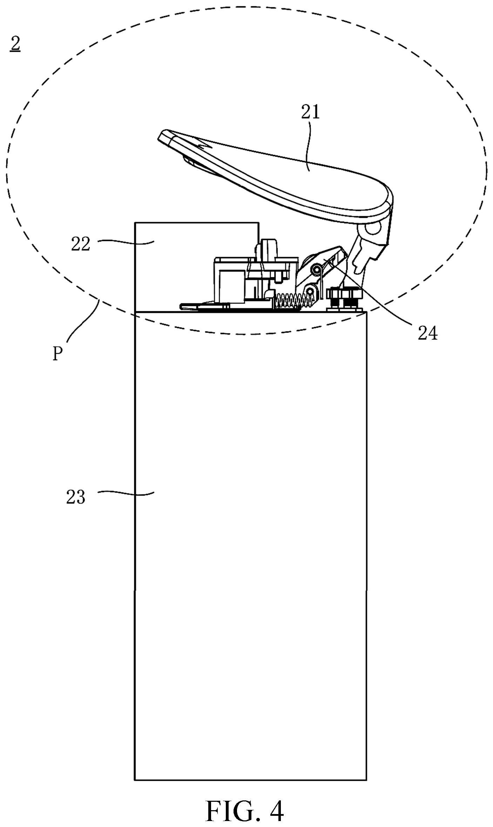

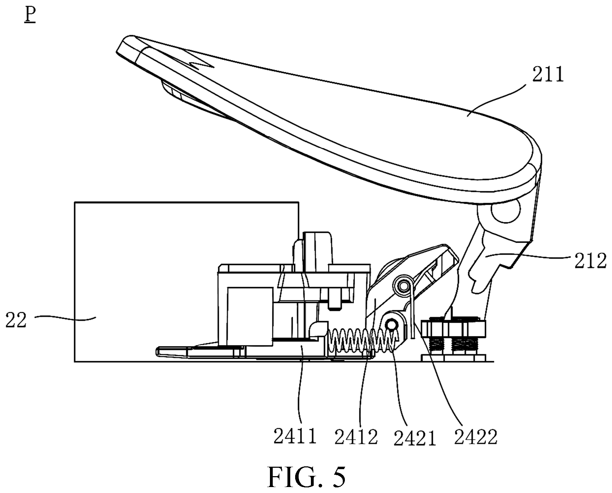

Refer to FIG. 4 and FIG. 5. During the cover-opening idle stroke, the flip cover 21 is decoupled from the limiting mechanism 24, and the limiting mechanism 24 remains in the limiting state.

For example, the limiting mechanism 24 includes a rotating assembly 241 and an elastic assembly 242. The elastic assembly 242 is connected to the rotating assembly 241. The rotating assembly 241 is configured to be rotatably connected to the housing 23. For example, the rotating assembly 241 is rotatably connected to the housing 23. The elastic assembly 242 is configured to be connected to the housing 23. For example, the elastic assembly 242 is connected to the housing 23.

During the cover-opening idle stroke, the flip cover 21 is decoupled from the rotating assembly 241, and the elastic assembly 242 drives the rotating assembly 241 to be in the limiting state, so that the rotating assembly 241 continues to limit the atomization mechanism 22 in the accommodating cavity. Because the flip cover 21 is decoupled from the rotating assembly 241, to cause the rotating assembly 241 to limit the atomization mechanism 22, in this case, an elastic force of the elastic assembly 242 applies an elastic force to the rotating assembly 241, and the elastic force causes the rotating assembly 241 to limit the atomization mechanism 22 in the accommodating cavity.

In some examples, the rotating assembly 241 includes only a rotating member 2412. The elastic assembly 242 includes only a third elastic member 2421.

In some other examples, the rotating assembly 241 includes a rotating member 2412 and a sliding member 2411. The rotating member 2412 is configured to be rotatably connected to the housing 23. For example, the rotating member 2412 is rotatably connected to the housing 23. The sliding member 2411 is configured to be slidably connected to the housing 23. For example, the sliding member 2411 is slidably connected to the housing 23 (for example, the housing 23 has a sliding groove, and the sliding member 2411 is slidably disposed in the sliding groove). The elastic assembly 242 includes a second elastic member 2422 and a third elastic member 2421. The second elastic member 2422 is connected to the rotating member 2412, and is configured to be connected to the housing 23. The third elastic member 2421 is connected to the sliding member 2411, and is configured to be connected to the housing 23. For example, both the second elastic member 2422 and the third elastic member 2421 are further connected to the housing 23. Descriptions are provided below by using an example in which the rotating assembly 241 includes the rotating member 2412 and the sliding member 2411, and the elastic assembly 242 includes the second elastic member 2422 and the third elastic member 2421.

During the cover-opening idle stroke, the flip cover 21 is decoupled from the rotating member 2412, and the third elastic member 2421 drives the sliding member 2411 to be in the limiting state. The rotating member 2412 is decoupled from the sliding member 2411, or the second elastic member 2422 drives the rotating member 2412 and the sliding member 2411 to be in the balanced state. In this way, because the rotating member 2412 is far away from the atomization mechanism 22, through engagement between the sliding member 2411 and the rotating member 2412, the limitation on the atomization mechanism 22 or release of the limitation is implemented, so that mounting of the atomization mechanism 22 and the limiting mechanism 24 has great fault tolerance, to better deploy the atomization mechanism 22 and the limiting mechanism 24, for example, a component such as a battery below, in the accommodating cavity. The disposed second elastic member 2422 acts on the rotating member 2412, and the disposed third elastic member 2421 acts on the sliding member 2411. The two operations have clear functional division and do not interfere with each other.

The sliding member 2411 may be a component capable of realizing the foregoing functions and performing sliding movement. Accordingly, a shape of the sliding member 2411 may be designed based on a requirement of the atomization apparatus 2. For example, the sliding member 2411 may be a structure such as a sliding rod or a sliding block. The rotating member 2412 is a component that is mounted on the housing 23 and can rotate relative to the housing 23. For example, the rotating member 2412 may be a structure such as a rod, an elongated plate, or an elongated strip.

The sliding member 2411 is engaged with the limiting portion of the atomization mechanism 22. In some examples, the sliding member 2411 is inserted in the limiting portion of the atomization mechanism 22, to limit the atomization mechanism 22 in the accommodating cavity. In this case, the sliding member 2411 may be a pin, and the limiting portion may be a groove 223; and in the limiting state, a part of the sliding member 2411 is inserted in the groove 223. In this way, the atomization mechanism 22 is limited in the accommodating cavity by using a simple insertion function. For example, an outer wall of the atomization assembly 221 has the groove 223. For another example, an outer wall of the atomization cartridge 222 has the groove 223. For another example, the groove 223 is formed between an outer bottom wall of the atomization assembly 221 and an outer top wall of the atomization cartridge 222. The second elastic member 2422 may be a torsion spring and/or a coil spring, and the third elastic member 2421 may be a spring and/or a spring sheet. In some other examples, the sliding member 2411 is snap-fitted to the limiting portion of the atomization mechanism 22, to limit the atomization mechanism 22 in the accommodating cavity. In this case, the limiting portion and the sliding member 2411 may be contact-type snaps. In some other examples, the sliding member 2411 is bonded to the limiting portion of the atomization mechanism 22, to limit the atomization mechanism 22 in the accommodating cavity. In this case, the limiting portion and the sliding member 2411 may be hook and loop fasteners, or the like.

The flip cover 21 includes a cover body 211 and a trigger portion 212, and the trigger portion 212 is connected to the cover body 211. For example, the cover body 211 and the trigger portion 212 are of an integral structure. The cover body 211 may be rotatably connected to the housing 23. In this way, when the cover body 211 is opened, the trigger portion 212 is rotated with the cover body 211, so that the trigger portion 212 is coupled to or decoupled from the rotating member 2412. During the cover-opening idle stroke, the flip cover 21 being decoupled from the limiting mechanism 24 means that both the cover body 211 and the trigger portion 212 are decoupled from the limiting mechanism 24.

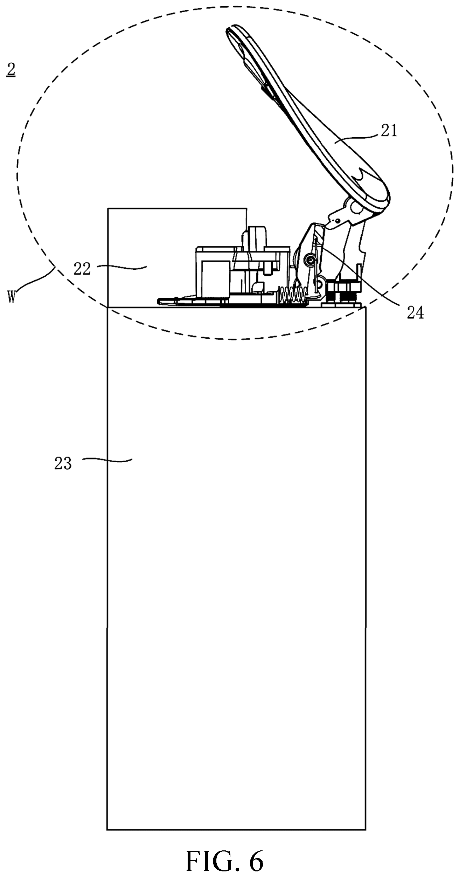

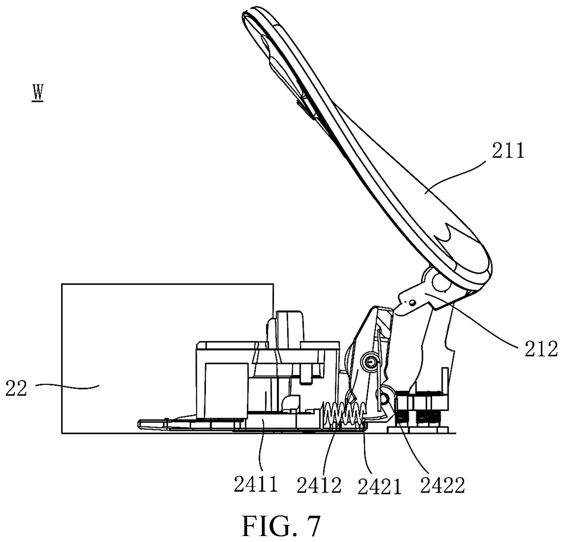

Refer to FIG. 6 and FIG. 7. During the cover-opening coupling stroke, the flip cover 21 is coupled to the limiting mechanism 24, so that the flip cover 21 switches the state of the limiting mechanism 24 to the released state (that is, switches from the limiting state to the released state).

For example, the limiting mechanism 24 includes a rotating assembly 241 and an elastic assembly 242. During the cover-opening coupling stroke, the flip cover 21 is coupled to the rotating assembly 241, to cause a state of the rotating assembly 241 to be switched to the released state, and the elastic assembly 242 forces the rotating assembly 241 to reset to the limiting state. In this way, through engagement between the rotating assembly 241 and the elastic assembly 242, the atomization mechanism 22 is limited, and the rotating assembly 241 is engaged with the cover plate to release the limitation on the atomization mechanism 22.

During the transition from the cover-opening idle stroke to the cover-opening coupling stroke, a position of the rotating assembly 241 changes, so that an elastic force of the elastic assembly 242 increases. Therefore, during the cover-opening coupling stroke, the elastic force of the elastic assembly 242 can restore the rotating assembly 241 to the state of limiting the atomization mechanism 22.

In some examples, the rotating assembly 241 includes a rotating member 2412 and a sliding member 2411. The elastic assembly 242 includes a second elastic member 2422 and a third elastic member 2421. During the cover-opening coupling stroke, the flip cover 21 is rotated to be coupled to the rotating member 2412, and the rotating member 2412 abuts against the sliding member 2411. The rotating member 2412 drives the sliding member 2411 to rotate to be in the released state. Both an elastic force of the second elastic member 2422 and an elastic force of the third elastic member 2421 increase. In this case, an increased elastic force of the second elastic member 2422 can force the sliding member 2411 to restore to the state of limiting the atomization mechanism 22; and an increased elastic force of the third elastic member 2421 can force the rotating member 2412 to restore.

In some examples, the flip cover 21 includes a cover body 211 and a trigger portion 212. During the cover-opening coupling stroke, the flip cover 21 being coupled to the limiting mechanism 24 means that the trigger portion 212 is coupled to the limiting mechanism 24, and the cover body 211 is decoupled from the limiting mechanism 24. In other words, the cover body 211 is coupled to the limiting mechanism 24 through the limiting portion.

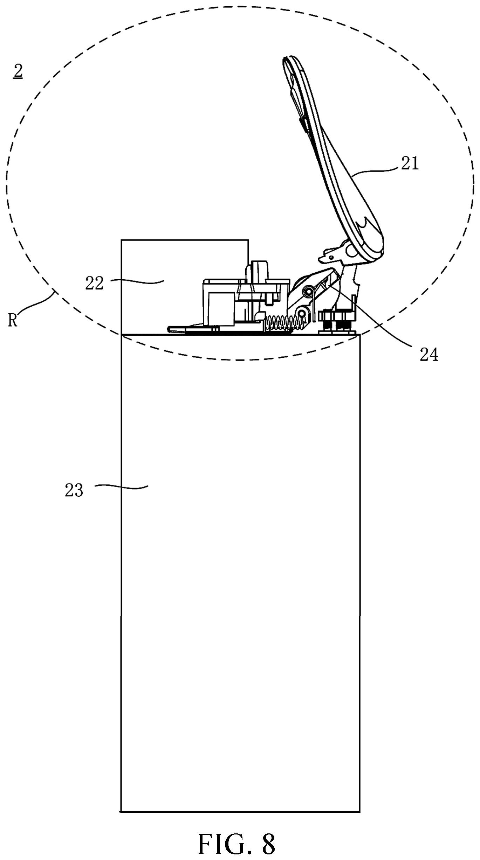

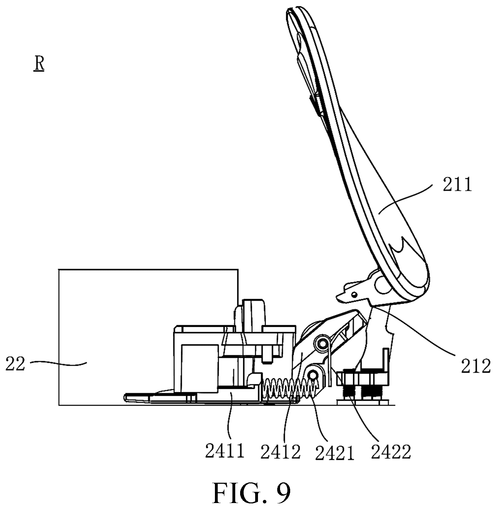

Refer to FIG. 8 and FIG. 9. During the cover-opening free stroke, the flip cover 21 is decoupled from the limiting mechanism 24. The limiting mechanism 24 remains in the released state. In this way, the flip cover 21 continues to be opened, so that the opening size of the mounting opening is large. Therefore, when the atomization mechanism 22 is removed, the atomization mechanism 22 does not come into contact with the flip cover 21. In this way, it is convenient to remove the atomization mechanism 22 from the accommodating cavity.

During the transition from the cover-opening coupling stroke to the cover-opening free stroke, the atomization mechanism 22 is not removed, in other words, the atomization mechanism is still located in the accommodating cavity. In this case, the limiting mechanism 24 is configured to abut against the non-limiting portion of the atomization mechanism 22. For example, because the first elastic member causes the atomization mechanism 22 to eject, the limiting mechanism 24 abuts against the non-limiting portion of the atomization mechanism 22, thereby reducing an ejection speed of the atomization mechanism 22.

For example, during the cover-opening free stroke, because an old atomization mechanism 22 is removed, a new atomization mechanism 22 is mounted. Because the atomization mechanism 22 is sprung up by the first elastic member, the limiting mechanism 24 abuts against the non-limiting portion of the atomization mechanism 22. In this case, in a subsequent cover-closing process, when the mounting opening is covered by using the flip cover, the atomization mechanism 22 can further be pressed, so that the atomization mechanism 22 is limited by the limiting mechanism.

For another example, during the cover-opening free stroke, because an old atomization mechanism 22 is removed, a new atomization mechanism 22 is mounted. In this case, the atomization mechanism 22 is manually pressed, so that the limiting mechanism 24 can be directly engaged with the limiting portion of the atomization mechanism 22, to limit the atomization mechanism 22 in the accommodating cavity.

During the cover-opening coupling stroke, the released state of the limiting mechanism 24 is that the limiting mechanism 24 is decoupled from the atomization mechanism 22. During the cover-opening free stroke, when the limiting mechanism 24 is in the released state, the limiting mechanism 24 is in contact with the non-limiting portion of the atomization mechanism 22. The non-limiting portion may be understood as an outer wall of the atomization mechanism 22, for example, an outer wall of the atomization cartridge 222, or an outer wall of the atomization assembly 221. The flip cover 21 includes a cover body 211 and a trigger portion 212. During the cover-opening free stroke, the flip cover 21 being decoupled from the limiting mechanism 24 means that both the trigger portion 212 and the cover body 211 are decoupled from the limiting mechanism 24.

For example, the limiting mechanism 24 includes a rotating assembly 241 and an elastic assembly 242. During the cover-opening free stroke, the flip cover 21 is decoupled from the rotating assembly 241, the limiting mechanism 24 remains in the released state, and the elastic assembly 242 drives the limiting mechanism 24 to abut against the non-limiting portion of the atomization mechanism 22.

In some examples, the rotating assembly 241 includes a rotating member 2412 and a sliding member 2411. The elastic assembly 242 includes a second elastic member 2422 and a third elastic member 2421. During the cover-opening free stroke, the flip cover 21 is decoupled from the rotating member 2412, and the third elastic member 2421 drives the sliding member 2411 to abut against the non-limiting portion of the atomization mechanism 22. The rotating member 2412 is decoupled from the sliding member 2411, or the second elastic member 2422 drives the rotating member 2412 and the sliding member 2411 to be in the balanced state.

The limiting mechanism 24 is configured to abut against the non-limiting portion of the atomization mechanism 22. In some examples, after the limitation on the atomization mechanism 22 is released, a liquid pushing mechanism 26 can be used to push the atomization mechanism 22 out of the accommodating cavity, so that a position of the limiting portion of the atomization mechanism 22 changes with the atomization mechanism 22.

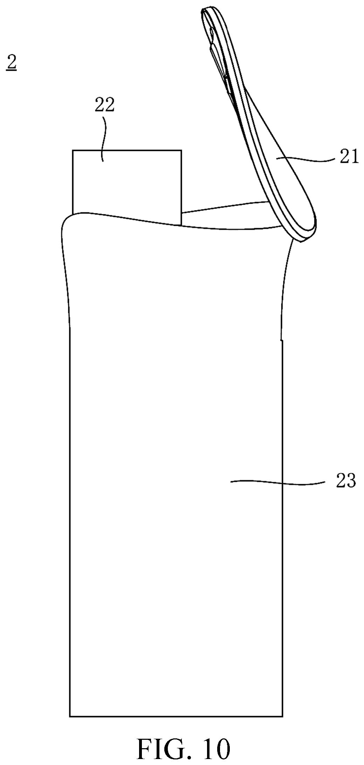

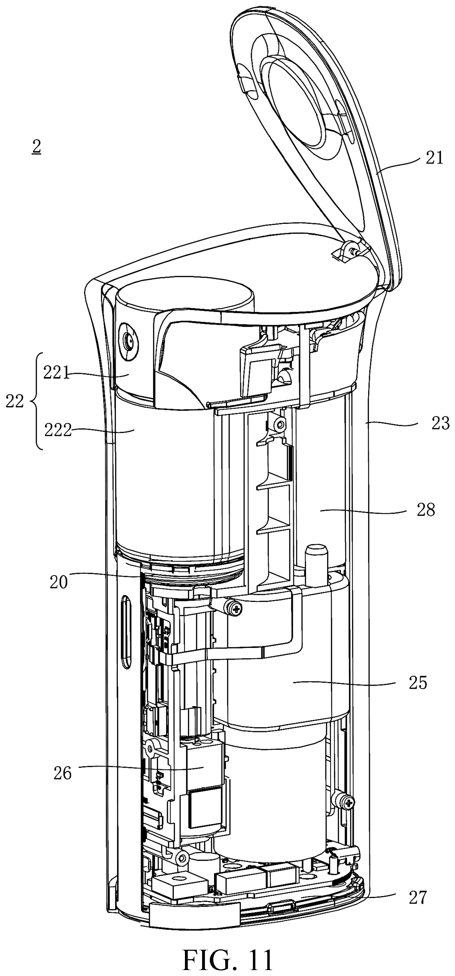

In some other examples, as shown in FIG. 10 and FIG. 11, the atomization apparatus further includes a first elastic member 20. The first elastic member 20 is configured to be disposed in the accommodating cavity. For example, the first elastic member 20 is disposed in the accommodating cavity. The atomization mechanism 22 is mounted in the accommodating cavity. Because the atomization mechanism 22 presses the first elastic member 20, a compressive elastic force is generated by the first elastic member 20. In other words, the first elastic member 20 is configured to apply an elastic force to the atomization mechanism 22. During the cover-opening coupling stroke, because the limiting mechanism releases the limitation on the atomization mechanism 22, the compressive elastic force of the first elastic member 20 makes the atomization mechanism 22 eject from the accommodating cavity, so that the position of the limiting portion of the atomization mechanism 22 changes, and it is convenient for an operator to take the atomization mechanism 22. In addition, the limiting mechanism 24 abuts against the non-limiting portion of the atomization mechanism 22, to reduce the ejection speed of the atomization mechanism 22, so as to prevent the atomization mechanism 22 from colliding with the flip cover 21, which causes damage to the atomization mechanism 22 and the flip cover 21. During the cover-opening free stroke, the limiting mechanism 24 (for example, the limiting mechanism 24) normally abuts against the limiting portion of the atomization mechanism 22. Because the position of the limiting portion changes, the limiting mechanism 24 can only abut against another part (that is, the non-limiting portion) of the atomization mechanism 22. In other words, during the cover-opening free stroke, the limiting mechanism 24 is configured to abut against the non-limiting portion of the atomization mechanism 22 by using the elastic force of the first elastic member 20. The first elastic member 20 may be at least one of a leaf spring, a spring, and an elastic sheet. Descriptions are provided below by using an example in which the position of the limiting portion of the atomization mechanism 22 can be changed by using the first elastic member 20.

In some embodiments, in the process of closing the flip cover 21, linkage engagement between the flip cover 21 and the limiting mechanism 24 is discontinuous coupling, to cause the flip cover 21 to switch the state of the limiting mechanism 24 from the released state to the limiting state.

In this way, for example, during the cover-opening free stroke, the position of the limiting portion of the atomization mechanism 22 changes. Therefore, when the flip cover 21 is closed, and the flip cover 21 can cover the mounting opening, the limiting mechanism 24 can limit the atomization mechanism 22 in the accommodating cavity, to omit the step of separately operating the limiting mechanism 24 to the atomization mechanism 22.

For example, in the process of closing the flip cover 21, the process of closing the flip cover 21 sequentially includes a cover-closing coupling stroke and a cover-closing idle stroke. For another example, in the process of closing the flip cover 21, the process of closing the flip cover 21 sequentially includes a cover-closing free stroke, a cover-closing coupling stroke, and a cover-closing idle stroke. The following describes the cover-closing free stroke, the cover-closing coupling stroke, and the cover-closing idle stroke of the flip cover 21 in detail.

During the cover-closing free stroke, the flip cover 21 is decoupled from the limiting mechanism 24, and the state of the limiting mechanism 24 is the released state.

When the atomization mechanism 22 is mounted in the accommodating cavity, for example, the limiting mechanism 24 abuts against the non-limiting portion of the atomization mechanism 22, so that the state of the limiting mechanism 24 is the released state. Therefore, an action of the cover-closing free stroke in the cover-closing process is opposite to an action of the cover-opening free stroke in the cover-opening process, with all other features being identical. For understanding of the cover-closing free stroke, refer to the related descriptions of the cover-opening free stroke. The flip cover 21 (for example, the cover body 211) may be decoupled from the atomization mechanism 22, that is, the cover-closing free stroke facilitates mounting of the atomization mechanism 22 in the housing 23, thereby avoiding interference of the flip cover 21. Certainly, the flip cover 21 may be in contact with the atomization mechanism 22.

During the cover-closing coupling stroke, the flip cover 21 is coupled to the limiting mechanism 24, and the state of the limiting mechanism 24 is the released state. The state of the limiting mechanism 24 being the released state during the cover-closing coupling stroke may be understood as the limiting mechanism 24 being separated from the atomization mechanism 22, or may be understood as the limiting mechanism 24 abutting against the non-limiting portion of the atomization mechanism 22.

In some examples, the rotating assembly 241 includes a rotating member 2412 and a sliding member 2411. The elastic assembly 242 includes a second elastic member 2422 and a third elastic member 2421. During the cover-closing coupling stroke, the flip cover 21 is coupled to the rotating member 2412, and the rotating member 2412 is decoupled from the sliding member 2411. The third elastic member 2421 drives the sliding member 2411 to abut against the non-limiting portion of the atomization mechanism 22, so that the state of the limiting mechanism 24 is the released state. The second elastic member 2422 is configured to drive the rotating member 2412 to reset to a state in which the rotating member 2412 just comes into contact with the sliding member 2411.

During the cover-closing idle stroke, the flip cover 21 is coupled to the limiting mechanism 24, and the flip cover 21 is configured to press the atomization mechanism 22, to cause the state of the limiting mechanism 24 to be switched to the limiting state.

An action of the cover-closing idle stroke in the cover-closing process is opposite to an action of the cover-opening idle stroke in the cover-opening process, with all other features being identical. Therefore, for understanding of the cover-closing idle stroke, refer to the related descriptions of the cover-opening idle stroke. However, the limiting mechanism 24 abuts against the non-limiting portion of the atomization mechanism 22. During the cover-closing idle stroke, the flip cover 21 is configured to press the atomization mechanism 22, to cause the limiting portion of the atomization mechanism 22 to reset (that is, the limiting portion is aligned with the limiting mechanism 24), so that the limiting mechanism 24 is engaged with the limiting portion (for example, the pin is inserted in the groove 223), and the state of the limiting mechanism 24 is switched to the limiting state.

In some embodiments, in the process of closing the flip cover 21, linkage engagement between the flip cover 21 and the limiting mechanism 24 is decoupling, and the state of the limiting mechanism 24 remains in the limiting state. In this way, the atomization mechanism 22 may be pressed manually or by other means, so that the limiting portion of the atomization mechanism 22 is engaged with the limiting mechanism 24, to limit the atomization mechanism 22 in the accommodating cavity. Then, the flip cover is closed, so that the flip cover 21 covers the mounting opening.

In the embodiments, the cover-closing process may alternatively include a cover-closing free stroke, a cover-closing coupling stroke, and a cover-closing idle stroke. For understanding of the cover-closing free stroke, the cover-closing coupling stroke, and the cover-closing idle stroke in the embodiments, refer to the related descriptions of the cover-closing free stroke, the cover-closing coupling stroke, and the cover-closing idle stroke in the foregoing embodiments. The cover-closing idle stroke in the embodiments is different from the cover-closing stroke in the foregoing embodiments. In other words, in the cover-closing idle stroke in the embodiments, the flip cover 21 does not press the atomization mechanism 22.

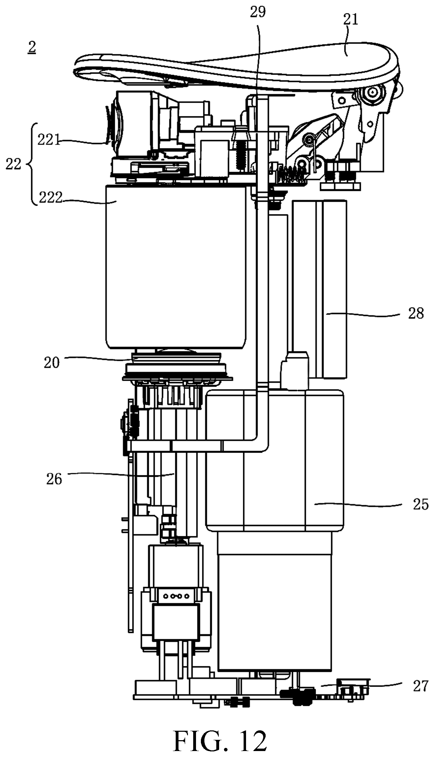

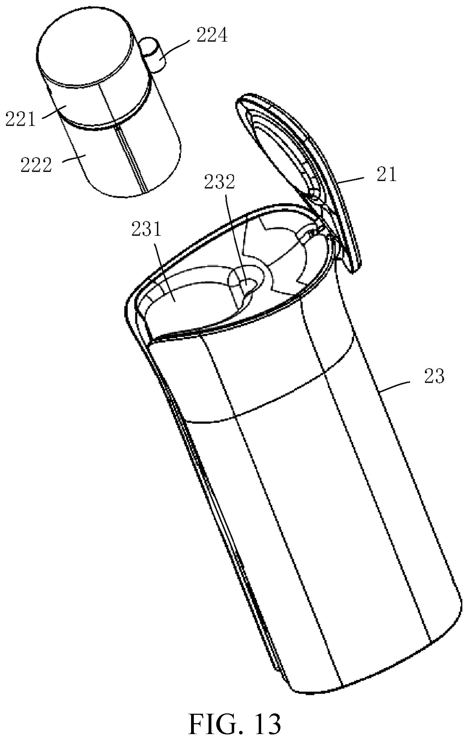

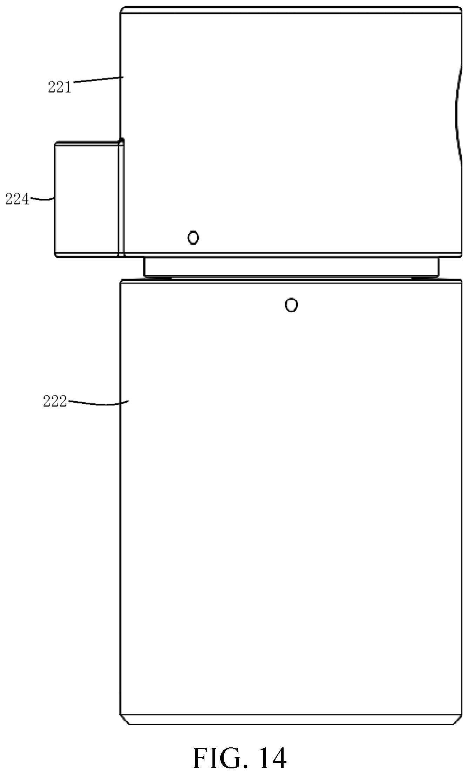

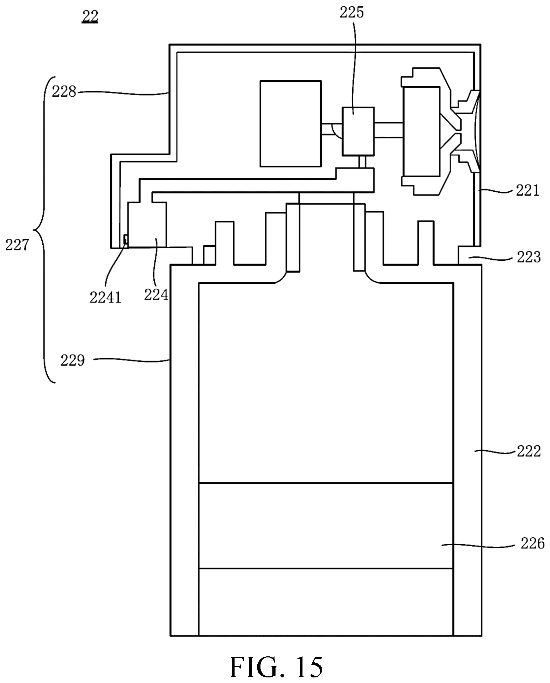

In some embodiments, refer to FIG. 11 to FIG. 15. FIG. 13 shows a structure of a part of the housing, to reflect an engagement relationship between the housing and an air supply interface. The atomization apparatus 2 further includes the air supply mechanism 25. The air supply mechanism 25 is configured to provide air for the atomization assembly 221. In this way, the air provided by the air supply mechanism 25 may enter the atomization mechanism 22, so that the liquid in the atomization mechanism 22 is atomized and sprayed. The air supply mechanism 25 may include an air pump and a vent tube. The air pump communicates with the atomization mechanism 22 (or the air supply interface 224) through the vent tube.

The air supply interface 224 protrudes from a first outer wall 227 of the atomization assembly 221, and the air supply mechanism 25 communicates with the air supply interface 224. The air supply interface 224 protrudes from the first outer wall 227 of the atomization mechanism 22, and may serve as a positioning and foolproof function.

The accommodating cavity 231 is provided with a positioning groove 232 engaged with the air supply interface 224. In this way, after the atomization mechanism 22 is mounted in the accommodating cavity 231, engagement between the air supply interface 224 and the positioning groove 232 can be used, so that the atomization mechanism 22 serves as a positioning and foolproof function, and a direction of a nozzle on the atomization mechanism 22 can also be positioned. In some examples, when the limiting mechanism 24 is engaged with the limiting portion of the atomization mechanism 22, the air supply interface 24 is also engaged with the positioning groove 232. For example, the mounting opening is provided at the top of the housing 23, and the mounting opening can extend from the top of the housing 23 in a height direction of the housing, to form the accommodating cavity 231 (that is, the accommodating cavity 231 may be referred to as an accommodating groove, and a groove opening of the accommodating groove may be the mounting opening). In this case, the top of the housing 23 further has the positioning groove 232 extending from the top of the housing 23 in the height direction of the housing.

An inner wall of the air supply interface 224 has a snap-fit groove 2241, and the air supply mechanism 25 has a protrusion. Alternatively, the inner wall of the air supply interface 224 has a protrusion, and the air supply mechanism 25 has a snap-fit groove. When the air supply mechanism 25 communicates with the air supply interface 224, the protrusion is snap-fitted in the snap-fit groove 2241. In other words, the air supply mechanism 25 (for example, the vent tube) is fixedly connected to and communicates with the air supply interface 224 by using a protrusion and snap-fit groove structure.

For example, the atomization mechanism 22 may include an atomization assembly 221 and an atomization cartridge 222. A second outer wall 228 of the atomization assembly 221 and a third outer wall 229 of the atomization cartridge 222 form the first outer wall 227, and the air supply interface 224 protrudes from the second outer wall 228. In some examples, the second outer wall 228 is located inside the third outer wall 229, in other words, an orthographic projection of the third outer wall 229 onto a first plane falls within an orthographic projection of the second outer wall 228 onto the first plane, and the first plane is perpendicular to a mounting direction of the atomization mechanism 22 (that is, a direction of the elastic force of the first elastic member 20). In some other examples, the second outer wall 228 is flush with the third outer wall 229. Therefore, the air supply interface 224 serves as a positioning and foolproof function.

The atomization cartridge 222 communicates with the atomization assembly 221, so that a liquid in the atomization cartridge 222 can flow into the atomization assembly 221. In this way, a path between the atomization cartridge 222 and the atomization assembly 221 is short, and a loss of liquid is correspondingly reduced.

The atomization cartridge 222 is detachably connected to the atomization assembly 221. For example, the atomization cartridge 222 is connected to the atomization assembly 221 via a snap-fit connection, a threaded connection, or the like. The atomization assembly 221 has a liquid supply inlet, the atomization cartridge 222 has a liquid supply outlet, and the liquid supply inlet and the liquid supply outlet are in detachable communication, to implement a detachable connection between the atomization assembly 221 and the atomization cartridge 222. Through the detachable connection, the atomization cartridge 222 or the atomization assembly 221 may be separately replaced, to reduce deterioration of the liquid in the atomization cartridge 222. In addition, for liquids in different atomization cartridges 222, different atomization assemblies 221 may be matched, to maximize absorption of the liquid.

The atomization assembly 221 has an atomization cavity 225, configured to receive the liquid from the atomization cartridge 222 and the air from the air supply mechanism 25, so that the nozzle of the atomization assembly 221 can spray an atomized liquid. The atomization cartridge 222 is configured to store a liquid.

The atomization cartridge 222 has a liquid storage cavity, configured to store a liquid.

The atomization apparatus 2 further includes a liquid pushing assembly. The liquid pushing assembly is configured to push the liquid in the atomization cartridge 222 into the atomization assembly 221.

The atomization assembly 221, the atomization cartridge 222, and the liquid pushing mechanism are sequentially arranged in an axial direction. In this way, in a structurally simple manner, the liquid pushing mechanism is caused to push the liquid in the atomization cartridge 222 into the atomization assembly 221. For example, an axial direction may be a height direction of the atomization apparatus. For another example, an axial direction may be a direction intersecting the height direction of the atomization apparatus, and an included angle between the axial direction and the height direction ranges from -10° to 10°.

The liquid pushing assembly may include a pushing rod, a threaded rod, a driving member, and a pressing plate. The driving member is connected to the threaded rod, and the threaded rod is threadedly connected to the pushing rod. The driving member is configured to drive the threaded rod to rotate, so that the pushing rod moves along an axis of the threaded rod, and the pushing rod pushes the liquid in the atomization cartridge 222 into the atomization assembly 221. In some examples, the driving member may be a motor, and a speed change mechanism is further provided. The driving member is in transmission connection with the speed change mechanism, and the speed change mechanism is connected to the threaded rod. The speed change mechanism may be a speed reducer, a transmission, or the like.

The pressing plate is disposed on the driving member. For example, the pressing plate is fastened onto the speed change mechanism. The pressing plate is rotatably connected to the threaded rod. In this way, the pushing rod can first press against the pressing plate, thereby preventing the pushing rod from damaging the driving member. In some examples, the pressing plate is fastened onto the driving member, and is sleeved onto an outer ring of a first bearing, and the threaded rod is sleeved in an inner ring of the first bearing. A piston 226 is disposed in the atomization cartridge 222. The liquid pushing mechanism 26 causes the liquid in the liquid storage cavity of the atomization cartridge 222 to flow into the atomization cavity 225 of the atomization assembly 221 through movement of the piston 226.

In some examples, the piston 226 is directly connected to the liquid pushing mechanism 26. For example, the piston 226 is connected to the liquid pushing mechanism 26 through a connecting member (which may be a plate, a rod, or the like). In some other examples, the piston 226 is indirectly connected to the liquid pushing mechanism 26. For example, the piston 226 can abut against the liquid pushing mechanism 26.

The pushing rod is sleeved in an inner ring of a second bearing. For example, an outer ring of the second bearing abuts against the piston 226. For another example, the outer ring of the first bearing is connected to the piston 226 through the connecting member. Therefore, it is possible to prevent the piston 226 from rotating while being driven to move by the pushing rod.

In some embodiments, the atomization apparatus 2 further includes a first sensor 29 and a controller. The first sensor 29 is electrically connected to the controller. The controller is configured to: when the first sensor 29 detects that the atomization cartridge 222 is mounted in the accommodating cavity 231, control the liquid pushing mechanism 26 to move toward the atomization cartridge 222, to reduce waiting time for liquid discharge of the atomization apparatus after startup. Certainly, when there is no atomization cartridge 222 in the accommodating cavity 231, the liquid pushing mechanism 26 is in an initial state (which may be understood as a movement amount of the liquid pushing mechanism 26 being 0 or close to 0), or returns to the initial state.

In some embodiments, the atomization apparatus 2 further includes a second sensor and a controller. The first sensor 29 is electrically connected to the controller. The controller is further configured to: when the second sensor detects that the liquid pushing mechanism 26 abuts against the piston 226, control the liquid pushing mechanism 26 to stop moving, or retract by a preset distance. Rapid liquid discharge is achieved after startup.

For example, a magnet is disposed on the piston 226, and when the sensor detects that the liquid pushing mechanism 26 comes into contact with the magnet, the controller controls the liquid pushing mechanism 26 to stop moving, or retract by the preset distance.

In some examples, the first sensor 29 and the second sensor may be Hall sensors. The first sensor 29 and the second sensor are mounted in the housing 23. The first sensor 29 and the second sensor may be a same sensor, or may be two sensors. An example in which the first sensor 29 and the second sensor are the same sensor (for example, the first sensor 29) is used for description in this specification.

The controller may be integrated on a circuit board 27. A switch may be mounted on the housing 23. The power supply 28 may be a secondary battery, for example, a lithium battery. The power supply 28 and the circuit board 27 may be mounted in the accommodating cavity 231 or the receiving cavity.

In some examples, the circuit board 27 is mounted on an inner bottom wall of the housing 23, and both the liquid pushing mechanism 26 and the air supply mechanism 25 are located on the circuit board 27. The first elastic member 20 is located on the liquid pushing mechanism 26. The atomization cartridge 222 is located on the first elastic member 20. The atomization assembly 221 is located on the atomization cartridge 222. The power supply 28 is located on the air supply mechanism 25. The limiting mechanism 24 is located on the power supply 28. In this way, the components are arranged compactly, so that a volume of the housing 23 is small, thereby achieving an objective of being compact and portable.

The foregoing descriptions are merely implementations of this application, and the protection scope of the patent of this application is not limited thereto. All equivalent structure or process changes made based on the content of the specification and the accompanying drawings in this application or by directly or indirectly applying this application in other related technical fields shall similarly fall within the protection scope of the patent of this application.

## Claims

1. A flip cover mechanism, comprising:
a flip cover; and
a limiting mechanism, having a limiting state and a released state, wherein
when the flip cover is rotated, linkage engagement between the flip cover and the limiting mechanism is discontinuous coupling, to cause the flip cover to switch a state of the limiting mechanism.

2. The flip cover mechanism of claim 1, wherein
in a process of opening the flip cover, linkage engagement between the flip cover and the limiting mechanism is discontinuous coupling, to cause the flip cover to switch the state of the limiting mechanism from the limiting state to the released state.

3. The flip cover mechanism of claim 2, wherein
the process of opening the flip cover sequentially comprises a cover-opening idle stroke and a cover-opening coupling stroke, wherein
during the cover-opening idle stroke, the flip cover is decoupled from the limiting mechanism, and the limiting mechanism remains in the limiting state; and
during the cover-opening coupling stroke, the flip cover is coupled to the limiting mechanism, to cause the flip cover to switch the state of the limiting mechanism to the released state.

4. The flip cover mechanism of claim 3, wherein
the limiting mechanism comprises a rotating assembly and an elastic assembly, wherein the elastic assembly is connected to the rotating assembly;
during the cover-opening idle stroke, the flip cover is decoupled from the rotating assembly, and the elastic assembly drives the rotating assembly to be in the limiting state; and
during the cover-opening coupling stroke, the flip cover is coupled to the rotating assembly, to cause the state of the rotating assembly to be switched to the released state, and the elastic assembly is configured to force the rotating assembly to reset to the limiting state.

5. The flip cover mechanism of claim 4, wherein
the rotating assembly comprises a rotating member and a sliding member; and the elastic assembly comprises a second elastic member and a third elastic member, wherein the second elastic member is connected to the rotating member, and the third elastic member is connected to the sliding member;
during the cover-opening idle stroke, the flip cover is decoupled from the rotating member, the third elastic member drives the sliding member to be in the limiting state, and the rotating member is decoupled from the sliding member, or the second elastic member drives the rotating member and the sliding member to be in a balanced state; and
during the cover-opening coupling stroke, the flip cover is coupled to the rotating member, to cause the rotating member to abut against the sliding member, the rotating member drives the sliding member to rotate to be in the released state, and both an elastic force of the second elastic member and an elastic force of the third elastic member increase.

6. The flip cover mechanism of claim 3, wherein
the process of opening the flip cover further comprises a cover-opening free stroke after the cover-opening coupling stroke, wherein
during the cover-opening free stroke, the flip cover is decoupled from the limiting mechanism, and the limiting mechanism remains in the released state.

7. The flip cover mechanism of claim 1, wherein
in a process of closing the flip cover, linkage engagement between the flip cover and the limiting mechanism is decoupling, and the state of the limiting mechanism remains in the limiting state.

8. An atomization apparatus, comprising:
a housing, having an accommodating cavity and a mounting opening;
an atomization mechanism, detachably mounted in the accommodating cavity; and
the flip cover mechanism according to any one of claims 1 to 7, wherein the flip cover is rotatably connected to the housing, and the flip cover is configured to cover the mounting opening; and the limiting mechanism is movably disposed in the housing.

9. The atomization apparatus of claim 8, wherein
the limiting mechanism is in insertion engagement with a limiting portion of the atomization mechanism; the limiting mechanism comprises a sliding member, and the limiting portion is a groove; and in the limiting state, at least a part of the sliding member is inserted in the groove.

10. The atomization apparatus of claim 9, wherein
the atomization mechanism comprises an atomization assembly and an atomization cartridge that are detachably connected; and an outer wall of the atomization assembly has a groove; or an outer wall of the atomization cartridge has a groove; or a groove is formed between an outer bottom wall of the atomization assembly and an outer top wall of the atomization cartridge.

11. The atomization apparatus of claim 8, wherein
the atomization apparatus further comprises a first elastic member, configured to apply an elastic force to the atomization mechanism, to cause the limiting mechanism to abut against a non-limiting portion of the atomization mechanism during the transition from the cover-opening coupling stroke to the cover-opening free stroke.
